# EUROPÄISCHE PATENTSCHRIFT

(11) **EP 4 313 596 B1**
(45) Veröffentlichungstag und Bekanntmachung des Hinweises auf die Patenterteilung: **06.08.2025**
(21) Anmeldenummer: 22716868.9
(22) Anmeldetag: 17.03.2022
(51) Int. Cl.: B32B 43/00, B32B 38/10, A61K 9/70

(54) **VERFAHREN ZUM HERSTELLEN EINES HAFTENDEN FOLIENVERBUNDS**
METHOD FOR PRODUCING AN ADHESIVE COMPOSITE FILM
PROCÉDÉ DE PRODUCTION D'UN FILM COMPOSITE ADHÉSIF

(30) Priorität: 23.03.2021 DE 102021107208
(43) Veröffentlichungstag der Anmeldung: 07.02.2024
(73) Patentinhaber: LTS LOHMANN Therapie-Systeme AG, 56626 Andernach (DE)
(72) Erfinder: STEINBORN, Peter, 56567 Neuwied (DE); WIEDERSBERG, Sandra, 06268 Steigra (DE); GUTEKUNST, Dorothea, 56410 Montabaur (DE); STEINBRECHER, Viktoria, 56626 Andernach (DE)
(74) Vertreter: dompatent von Kreisler Selting Werner - Partnerschaft von Patent- und Rechtsanwälten mbB
(86) Internationale Anmeldenummer: PCT/EP2022/057074
(87) Internationale Veröffentlichungsnummer: WO 2022/200187

(56) Entgegenhaltungen:
- EP-B1- 2 118 669
- EP-B1- 2 611 430
- US-B2- 6 684 925
- US-B2- 6 902 643

## Beschreibung

Die vorliegende Erfindung betrifft ein Verfahren zum Herstellen eines haftenden Folienverbunds für ein transdermales therapeutisches System (TTS).

Zur Herstellung von Folienprodukten, wie insbesondere transdermalen therapeutischen Systemen (TTS), werden häufig Laminate, vorzugsweise als Zwischenprodukte, benutzt. Ein derartiges Laminat weist insbesondere eine erste Folie, z.B. eine Trägerfolie, eine zweite Folie, z.B. eine Abdeckfolie, sowie eine zwischen der ersten und der zweiten Folie angeordnete Formulierung auf. Bei der Formulierung handelt es sich vorzugsweise um eine Matrix. Die Formulierung weist generell einen Klebstoff sowie vorzugsweise mindestens einen Wirkstoff und/oder weitere Hilfsstoffe auf.

Zur Weiterverarbeitung eines Laminats ist es meist notwendig eine der Folien, insbesondere die zweite Folie, von dem Laminat zu entfernen. Beispielsweise kommt es vor, dass das Laminat in einem Herstellungsschritt zu einer Laminatrolle aufgerollt wird, wobei eine der Folien, vorzugsweise die zweite Folie, als eine Zwischen- und/oder Schutzfolie dient. So kann es beispielsweise zum Aufrollen notwendig sein, die auf der Trägerfolie angeordnete Formulierung durch eine derartige Abdeckfolie zu schützen. Zur Weiterverarbeitung, insbesondere mittels einer anderen Maschine und/oder an einem anderen Ort und/oder zu einem anderen Zeitpunkt, kann es sodann notwendig sein, diese Abdeckfolie zu entfernen. Dieses Entfernen der Abdeckfolie erfolgt insbesondere nach einem Abrollen der Laminatrolle. Vorzugsweise erfolgt das Entfernen der Folie durch ein Abziehen.

Bei dem Entfernen der Abdeckfolie kommt es zu zahlreichen Problemen. So kommt es beispielsweise vor, dass bei einem Entfernen der Abdeckfolie Reste der Formulierung auf der Abdeckfolie verbleiben und/oder es durch das Entfernen zu einer ungleichmäßigen Verteilung der Formulierung kommt. Auch kann es zu einem ungleichmäßigen Abheben der Formulierung, insbesondere der wirkstoffhaltigen Klebermatrix kommen. Dies kann sich u. a. negativ auf eine Wirkstoffdosierung auswirken. Insbesondere kann es dazu kommen, dass eine Weiterverarbeitung der Matrix unmöglich ist. Diese Probleme liegen insbesondere vor, wenn es zu einer Veränderung der Haftkräfte zwischen Formulierung und Abdeckfolie, beispielsweise durch Temperatureinflüsse, besondere Beschaffenheiten der Formulierung oder eine Veränderung der Hafteigenschaften der Abdeckfolie, beispielsweise ein Abbau von Silikon bei einer silikonisierten Abdeckfolie, kommt. Ferner kommt es zu Problemen, wenn Folienmaterialien mit ähnlichen Haftungseigenschaften gewählt werden.

EP 2 611 430 B1 beschreibt: Ein Verfahren zum Herstellen eines transdermalen therapeutischen Systems mit Schritten zum Aufbringen eines bahnförmigen Wirkstoffdepots auf eine bahnförmige Trägerfolie, so dass das Wirkstoffdepot haftklebend an der Trägerfolie angebracht ist, zum Durchtrennen des bahnförmigen Wirkstoffdepots entlang linienförmiger Geometrien zur Abgrenzung von zumindest mehreren ersten Wirkstoffdepotabschnitten, zum Einbringen eines Schlitzes in die bahnförmige Trägerfolie von deren nicht mit dem Wirkstoffdepot belegten Seite, wobei der Schlitz zwei in Längsrichtung der Trägerfolie nebeneinander angeordnete Trägerfolienstreifen so definiert, dass die ersten Wirkstoffabschnitte jeweils auf beiden Trägerfolienstreifen aufliegen, zum Bewegen der geschlitzten Trägerfolie entlang deren Längsrichtung und Umlenken von einem ersten der Trägerfolienstreifen an einer ersten Umlenkeinrichtung in Richtung weg von der mit dem Wirkstoffdepot belegten Seite der Trägerfolie, und zum Zuführen eines Schutzfolienstreifens nach dem Umlenken des ersten Trägerfolienstreifens so, dass der Schutzfolienstreifen parallel zum zweiten Trägerfolienstreifen angeordnet die nicht vom zweiten Trägerfolienstreifen bedeckten Bereiche der auf diesem befindlichen ersten Wirkstoffdepotabschnitte bedeckt. Aufgabe der Erfindung ist es, ein Verfahren zum Herstellen eines haftenden TTS-Folienverbunds, zu schaffen, wobei das Entfernen einer Folie von einem Laminat verbessert ist.

Die Lösung der Aufgabe erfolgt erfindungsgemäß durch ein Verfahren zum Herstellen eines haftenden Folienverbunds nach Anspruch 1.

Bei dem erfindungsgemäßen Verfahren handelt es sich um ein Verfahren zum Herstellen eines TTS-Folienverbunds. Der herzustellende Folienverbund weist insbesondere mindestens eine Folie sowie eine einen Wirkstoff und/oder Klebstoff aufweisende Formulierung auf. Haftend meint insbesondere klebend. Bei Herstellung eines TTS-Folienverbunds ist es bevorzugt, dass der Folienverbund mindestens eine Trägerfolie sowie eine darauf angeordnete Matrix aufweist. Ein erster Schritt des Verfahrens besteht im Bereitstellen eines Laminats. Bei dem Laminat handelt es sich insbesondere um ein TTS-Laminat. Es ist bevorzugt, dass es sich bei dem bereitzustellenden Laminat um eine Laminatbahn, vorzugsweise eine Laminatfolienbahn, handelt. Eine bevorzugt alternative Bezeichnung für Laminatbahn ist Laminatband. Insbesondere erfolgt das Bereitstellen des Laminats von einer Laminatrolle. Das Laminat, insbesondere die Laminatbahn, wird bevorzugt aufgewickelt bereitgestellt. Die Laminatbahn hat vorzugsweise eine Länge von mindestens 1 m, bevorzugt mindestens 5 m, besonders bevorzugt mindestens 10 m. Vorzugsweise weist die Laminatbahn ein Verhältnis zwischen Länge zu Breite von mindestens 2:1, bevorzugt mindestens 5:1, besonders bevorzugt mindestens 10:1 auf. Das Laminat weist eine erste Folie, eine zweite Folie und eine zwischen der ersten Folie und der zweiten Folie angeordnete, haftende Formulierung auf. Bei der ersten Folie handelt es sich insbesondere um eine Trägerfolie, auch Intermediate Liner oder Release Liner genannt. Es ist bevorzugt, dass die erste Folie PET aufweist, insbesondere daraus besteht. Bei der zweiten Folie handelt es sich vorzugsweise um eine Abdeckfolie, auch Backing Layer genannt. Die zweite Folie weist vorzugsweise Papier und/oder Polyethylen auf, besteht insbesondere daraus. Besonders bevorzugt handelt es sich bei der zweiten Folie um eine Papierfolie, die mit Polyethylen beschichtet ist. Insbesondere ist die erste und/oder die zweite Folie silikonisiert. Die Formulierung weist einen Wirkstoff und/oder Klebstoff auf. Ein zweiter Schritt des Verfahrens besteht in dem Zuführen des Laminats in einer Zuführrichtung. Zuführen meint hierbei insbesondere ein Führen des Laminats zu einem Trennbereich, in dem die Folien des Laminats getrennt werden. Ein weiterer Schritt besteht in dem Trennen der ersten Folie mit der darauf angeordneten Formulierung von der zweiten Folie. Das Trennen erfolgt bevorzugt in einem Trennbereich. Es handelt sich insbesondere um ein abziehendes Trennen, so dass die erste Folie von der zweiten Folie abgezogen wird. Es ist hierbei bevorzugt, dass das Trennen derart erfolgt, dass nach dem Trennen die Formulierung auf der ersten Folie angeordnet ist, wobei es besonders bevorzugt ist, dass nach dem Trennen die zweite Folie im Wesentlichen formulierungsfrei ist. Das Trennen der ersten Folie von der zweiten Folie erfolgt durch ein schräg zur Zuführrichtung verlaufendes Abführen der ersten Folie von der zweiten Folie. Die zweite Folie wird hierbei in der Zuführrichtung weitergeführt. Ferner erfolgt beim Abführen der ersten Folie eine Drehung der ersten Folie um die eigene Längsachse (der ersten Folie). Das Abführen der ersten Folie erfolgt in einer Abführrichtung. Beim Abführen der ersten Folie schräg zur Zuführrichtung ist es bevorzugt, dass die erste Folie von der zweiten Folie und/oder der Zuführrichtung abknickt. Mit anderen Worten erfolgt das Trennen der ersten und der zweiten Folie durch ein Umfalten der ersten Folie im Trennbereich, insbesondere um 90°. Dieses Umfalten kann mit anderen Worten insbesondere beschrieben werden in Anlehnung an etwa ein Umfalten eines DIN-A4-Papiers, wobei eine Ecke des DIN-A4-Papiers nach innen über den Rand des Papiers hinaus gefaltet wird. Bevorzugt ist es, dass die Faltkante einen 45°-Winkel aufweist. Die Abführung erfolgt sodann in Richtung derjenigen kurzen Seite des DIN-A4-Blatts, die die vorstehend genannte Ecke des DIN-A4-Blatts aufweist. Es ist bevorzugt, dass es sich bei der abgeführten ersten Folie mit darauf angeordneter Formulierung um den herzustellenden Folienverbund handelt. Bevorzugt erfolgt das Verfahren als Endlosverfahren.

Überraschenderweise liegt durch die schräge, insbesondere im Winkel von 90°, Abführung der ersten Folie mit darauf angeordneter Formulierung Vorteil, dass bei der Trennung die Formulierung im Wesentlichen vollständig an der ersten Folie haftet und somit im Wesentlichen keinerlei Formulierung auf der zweiten Folie verbleibt.

In bevorzugter Ausführung erfolgt die schräge Abführung der ersten Folie in einem Winkel, auch Abführwinkel genannt, von über 70°, bevorzugt von 70° - 110°, besonders bevorzugt von ca. 90°, relativ zur Zuführrichtung. Wird die erste Folie im besonders bevorzugten Winkel von 90° abgeführt, ist es somit bevorzugt, dass die Abführrichtung der ersten Folie senkrecht zur Zuführrichtung des Laminats liegt, bzw. die erste abgeführte Folie senkrecht zum Laminat ist.

Es ist bevorzugt, dass die Drehung der ersten Folie um die eigene Längsachse um mindestens 60°, bevorzugt um 90° - 270° und besonders bevorzugt um ca. 180°, erfolgt. Die Drehung um 180° kann mit anderen Worten derart beschrieben werden, dass die Folien vor der Drehung frontseitig angeordnet ist, während sie nach der Drehung rückwärtig ist, besonders bevorzugt ist vor der Drehung eine Vorderseite der ersten Folie oben, während nach der Drehung die Rückseite der ersten Folie oben ist.

In bevorzugter Ausführung erfolgt das Zuführen des Laminats in einer Zuführebene, das Abführen der ersten Folie in einer Abführebene sowie ein vorzugsweises Weiterführen der zweiten Folie nach dem Trennen in einer Weiterführebene. Es ist bevorzugt, dass mindestens zwei der Zuführebene, der Weiterführebene und der Abführebene parallel, insbesondere identisch, zu einander sind. Mit anderen Worten werden mindestens zwei der folgenden Elemente parallel, insbesondere identisch, geführt: Laminat, abgeführte erste Folie und weitergeführte zweite Folie. Besonders bevorzugt ist es, dass das Laminat und die weitergeführte zweite Folie in identischer Ebene sowie in identischer Richtung geführt werden.

Die Formulierung weist einen Wirkstoff und/oder einen Klebstoff auf. Der Wirkstoff weist insbesondere einen transdermalen Wirkstoff. Der Klebstoff weist insbesondere einen Hautklebstoff auf.

In bevorzugter Ausführung ist zumindest eine der Folien haftreduziert. Diese Haftreduzierung ist insbesondere derart umgesetzt, dass zumindest eine der Folien silikonisiert ist, also mit Silikon beschichtet ist.

Einerseits ist es möglich, dass die erste und die zweite Folie eine im Wesentlichen gleiche Haftung aufweist und/oder, dass die Adhäsion der Matrix zu den beiden Folien im Wesentlichen gleich ist. Diese gleiche Haftung meint insbesondere eine gleiche Haftung bzw. Adhäsion zwischen der zweiten Folie und der Formulierung verglichen mit der Haftung bzw. Adhäsion zwischen der ersten Folie und der Formulierung. Andererseits ist es bevorzugt möglich, dass die zweite Folie eine geringere Haftung als die erste Folie aufweist. Mit anderen Worten weist hiernach die zweite Folie eine größere Haftreduzierung als die erste Folie auf. Diese geringere Haftung meint insbesondere eine geringere Haftung bzw. Adhäsion zwischen der zweiten Folie und der Formulierung bzw. eine größere geringere Haftung bzw. Adhäsion zwischen der ersten Folie und der Formulierung. Durch die unterschiedliche Haftung der Folien ist es insbesondere vorteilhaft umgesetzt, dass die Formulierung, insbesondere beim Trennen der Folien, an der ersten Folie haftet, während sie sich von der zweiten Folie löst.

Bevorzugt ist es, dass das Zuführen des Laminats durch ein Ziehen, insbesondere ein Aufrollen, der zweiten Folie erfolgt. Besonders bevorzugt ist es hierbei, dass zunächst das gesamte Laminat gezogen wird, wobei nach dem Trennen der ersten Folie mit darauf angeordneter Formulierung vom Laminat nur die zweite Folie weitergezogen wird. Dieses Ziehen erfolgt vorzugsweise mittels Zughaspel. Es ist bevorzugt, dass das Abführen der ersten Folie mittels Ziehens, insbesondere durch Aufrollen, der ersten Folie erfolgt. Besonders bevorzugt erfolgt das Ziehen mittels Zughaspel. Während zum Ziehen des Laminats bzw. der zweiten Folie sowie zum Ziehen der ersten Folie Zughaspeln genutzt werden, ist es bevorzugt, dass die Zugrichtungen der Zughaspel senkrecht zueinander und/oder in identischer Ebene liegend sind.

In bevorzugter Ausführung erfolgt das Abführen der ersten Folie entlang eine, vorzugsweise eckigen, Biegekante.

Es ist bevorzugt, dass die Drehung der ersten Folie um die eigene Längsachse durch ein Biegen der Folie an der Biegekante erfolgt.

Nachfolgend wird die Erfindung anhand bevorzugter Ausführungsformen unter Bezugnahme auf die anliegenden Zeichnungen näher erläutert.

Es zeigen:
- Fig. 1a: eine schematische Seitenansicht eines Laminats,
- Fig. 1b: eine schematische Explosionsansicht eines Laminats,
- Fig. 2a: eine schematische Seitenansicht eines Folienverbunds,
- Fig. 2b: eine schematische Explosionsansicht eines Folienverbunds,
- Fig. 3: eine schematische Draufsicht einer für das Verständnis der Erfindung nützlichen Ausführungsform einer Vorrichtung zum Herstellen eines haftenden Folienverbunds, und
- Fig. 4: eine schematische, perspektivische Ansicht einer weiteren für das Verständnis der Erfindung nützlichen Ausführungsform einer Vorrichtung zum Herstellen eines haftenden Folienverbunds.

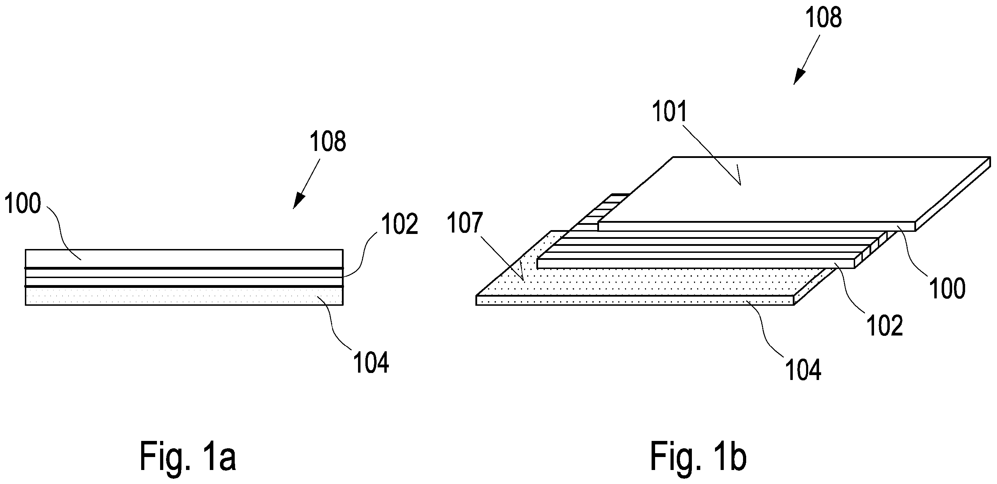

Fig. 1a zeigt beispielhaft eine Art eines standardmäßigen Laminats 108, welches als Ausgangsbasis für das erfindungsgemäße Verfahren zum Herstellen eines haftenden Folienverbunds 106 genutzt werden kann.

Das dargestellte Laminat 108 weist eine erste Folie 100, eine zweite Folie 104 sowie eine zwischen der ersten Folie 100 und der zweiten Folie 104 angeordnete Formulierung 102 auf. Bevorzugt handelt es sich bei dem Laminat 108 um ein TTS-Laminat. Bei der ersten Folie 100 handelt es sich insbesondere um eine Trägerfolie. Bei der zweiten Folie 104 handelt es sich vorzugsweise um eine Abdeckfolie. Die Formulierung 102 ist bevorzugt eine Matrix, die besonders bevorzugt einen Klebstoff sowie einen transdermalen Wirkstoff aufweist. Alternative Ausführungen eines Laminats 108 können weitere Schichten, wie beispielsweise weitere Folien oder Formulierungen, aufweisen.

Fig. 1b zeigt eine explodierte Darstellung des Laminats 108 aus Fig. 1a.

Die erste Folie 100 weist eine Oberfläche 101 mit einer ersten Hafteigenschaft auf, während die zweite Folie 104 eine Oberfläche 107 mit einer zweiten Hafteigenschaft aufweist. Bevorzugt ist es, dass sich die erste Hafteigenschaft der ersten Folie 100 von der zweiten Hafteigenschaft der zweiten Folie 104unterscheidet. Besonders bevorzugt ist es, dass die erste Folie 100 eine größere Haftung als die zweite Folie 104 aufweist. Es ist bevorzugt, dass die erste Folie 100 und/oder die zweite Folie 104 haftreduziert ist, insbesondere die erste Folie 100 eine geringe Haftreduzierung als die zweite Folie 104aufweist. Haftung meint hierbei insbesondere eine Adhäsionskraft zwischen der ersten Folie 100 und der Formulierung 102 bzw. der zweiten Folie 104und der Formulierung 102. Besonders bevorzugt ist die Adhäsionskraft zwischen der ersten Folie 100 und der Formulierung 102 größer als die Adhäsionskraft zwischen der zweiten Folie 104und der Formulierung 102. Durch diese unterschiedlichen Haftungen der ersten Folie 100 an der zweiten Folie 104 ist es vorzugsweise vorteilhaft, dass bei einem Trennen der Folien 100; 104 die Formulierung 102 an der ersten Folie 100 haftet und somit auf dieser verbleibt.

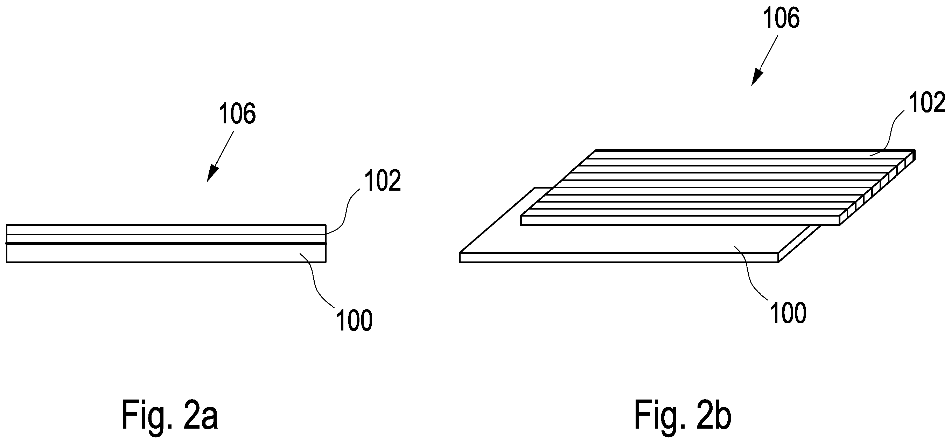

Fig. 2a zeigt beispielhaft einen Folienverbund 106, bei dem es sich vorzugsweise um ein mit dem erfindungsgemäßen Verfahren und/oder einer Vorrichtung herzustellenden Folienverbund 106 handelt.

Im Wesentlichen entspricht der Folienverbund 106 dem Laminat 108 aus Fig. 1a, wobei die zweite Folie 104 entfernt bzw. hiervon getrennt, insbesondere abgezogen wurde. Darüber hinaus wurde, verglichen mit der Ausführung aus Fig. 1a, eine Drehung um die eigene Längsachse, hier um 180°, vorgenommen. Dargestellt liegt nun demnach die erste Folie 100 unten, wobei die Formulierung 102 über der ersten Folie 100 angeordnet, insbesondere haftend mit dieser verbunden, ist.

Bei einer späteren Verwendung kann beispielsweise die Formulierung 102 zur transdermalen Darreichung auf einer Haut angeordnet werden, wobei vorzugsweise die erste Folie 100 die Formulierung zur anderen Seite verdeckt.

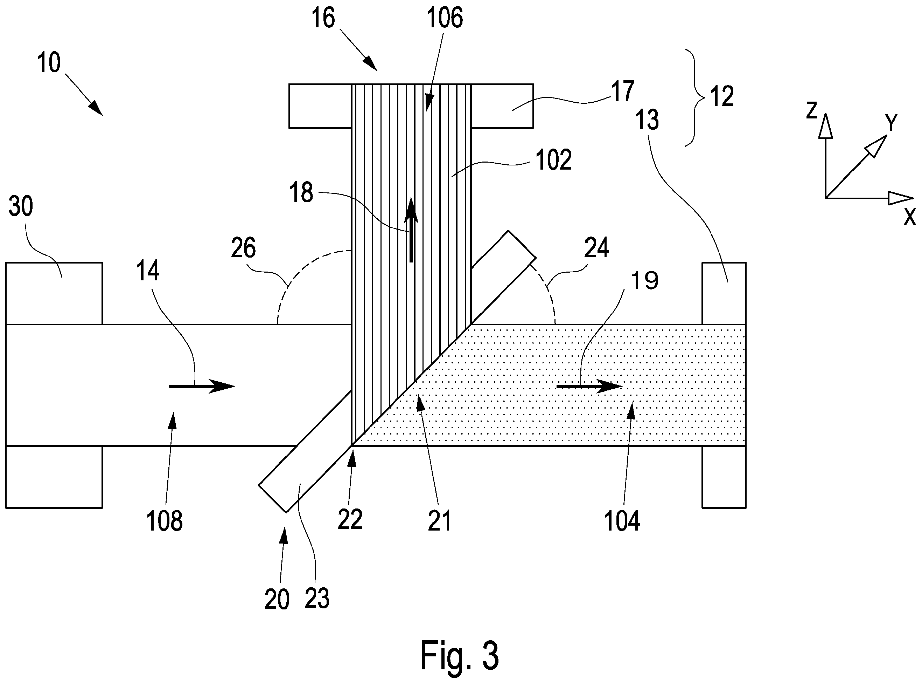

Fig. 3 zeigt eine Vorrichtung 10 zum Herstellen eines haftenden Folienverbunds 106. Bevorzugt handelt es sich bei dem dargestellten Folienverbund 106 um den in Fig. 2a dargestellten Folienverbund 106.

Auf einer Ablaufhaspel 30 wird ein Laminat 108 bereitgestellt. Bevorzugt handelt es sich bei dem Laminat 108 und das in Fig. 1a dargestellte Laminat 108.

Die Zuführung des Laminats 108 in Zuführrichtung (dargestellt durch Pfeil 14) erfolgt durch Laminatfördermittel 12. Dargestellt weist das Laminatfördermittel 12 zwei Zughaspeln 13; 17 auf. Die erste Zughaspel 17 wickelt den herzustellenden haftenden Folienverbund 106, bestehend aus Folie 100 mit darauf angeordneter Formulierung 102, auf. Die erste Zughaspel 17 ist demnach ebenfalls Teil des Folienverbundfördermittels 16 zur Förderung der ersten Folie 100 und darauf angeordneter Formulierung 102. Die zweite Zughaspel 13 wickelt die zweite Folie 104 des Laminats 108 auf. Durch das Ziehen der Folien 100; 104 des Laminats 108 mittels Zughaspeln 13; 17 erfolgt eine Zuführung des Laminats 108, durch die das Laminat 108 von der Ablaufhaspel 30 abgewickelt wird.

In alternativer, nicht dargestellter, Ausführungsform ist es beispielsweise möglich, lediglich das erste Zughaspel 17 als Laminatfördermittel 12 sowie als Folienverbundfördermittel 16 einzusetzen. Insbesondere würde das zweite Haspel somit in dieser alternativen Ausführungsform entfallen. Da es sich bei der zweiten Folie 104 häufig um ein Zwischenprodukt bzw. Abfallprodukt handelt, könnte dieses somit ohne Aufwicklung ausgeworfen werden. Bei dieser alternativen Ausführungsform entspräche somit das Laminatfördermittel 12 dem Folienverbundfördermittel 16, wobei das Laminatfördermittel 12 bzw. das Folienverbundfördermittel 16 das erste Zughaspel 17 aufweist.

Dargestellt in Fig. 3 fördert die erste Zughaspel 17 die erste Folie 100 in Abführrichtung 18. Die zweite Zughaspel 13 fördert die zweite Folie 104 in Weiterführrichtung 19. Bevorzugt ist es, dass mindestens einer, besonders bevorzugt alle der folgenden Gegebenheiten zutreffen: Zuführrichtung 14 ist parallel, vorzugsweise identisch zu Weiterführrichtung 19; Abführrichtung 18 liegt schräg, insbesondere im 90°-Winkel zur Zuführrichtung 14 und/oder zu Weiterführrichtung 19; der Zuführweg des Laminats 108 ist deckungsgleich zum Weiterführweg der zweiten Folie 104; die Abführung der ersten Folie 100 erfolgt in einer Abführebene (dargestellt in der xy-Ebene), wobei diese Ebene parallel, insbesondere identisch, zur Zuführebene des Laminats 108 und/oder zur Weiterführebene der zweiten Folie 104 ist.

In dargestellter Ausführung erfolgt in einem Trennbereich 21 ein, vorzugsweise abziehendes, Trennen der ersten Folie 100 mit darauf angeordneter Formulierung 102 von der zweiten Folie 104 des Laminats 108. Hierbei wird die erste Folie 100 um eine Biegekante 22 einer Trennvorrichtung 20 gebogen. In dargestellter Ausführung weist die Trennvorrichtung 20 insbesondere ein Biegeblech 23 auf, welches die Biegekante 22 ausbildet. Die Biegekante weist einen Biegewinkel 24 auf, der vorzugsweise 45° zur Zuführrichtung 14 und/oder 45° zur Weiterführrichtung 19; und/oder 45° zum zugeführten Laminat 108 und/oder 45° zur weitergeführten zweiten Folie 104 aufweist.

An der Biegekante 22 wird die erste Folie 100 des Laminats 108 derart umgefaltet bzw. umgeklappt, dass nach der Trennung die Unterseite der ersten Folie 100 mit darauf angeordneter Formulierung 102 nach oben weist. Somit erfolgte eine Drehung der ersten Folie 100 bei der Trennung um 180° um die eigene Längsachse der Folie 100. Die Abführung der ersten Folie 100 mit darauf angeordneter Formulierung 102 nach der Trennung erfolgt in Abführrichtung 18 in einem Abführwinkel 26. Der Abführwinkel 26 beträgt bevorzugt 90° zur Zuführrichtung 14 und/oder Weiterführrichtung 1 und/oder dem zugeführten Laminat 108 und/oder der abgeführten zweiten Folie 104.

Die Trennkante 22 ist vorzugsweise parallel zur Zuführebene des Laminats 108 und/oder zur Weiterführebene der zweiten Folie 104 und/oder zur Abführebene der ersten Folie 100 angeordnet. Besonders bevorzugt ist es, dass die Biegekante 22 derart angeordnet ist, dass das zugeführte Laminat 108 gegenüberliegend benachbart geführt, insbesondere berührend geführt, wird.

Überraschenderweise ergibt sich durch die schräge Abführung der ersten Folie 100 mit darauf angeordneter Formulierung 102, insbesondere im 90°-Abführwinkel 26, dass bei der Trennung die Formulierung 102 im Wesentlichen vollständig an der ersten Folie 100 haftet und somit im Wesentlichen keinerlei Formulierung 102 auf der zweiten Folie 104 verbleibt.

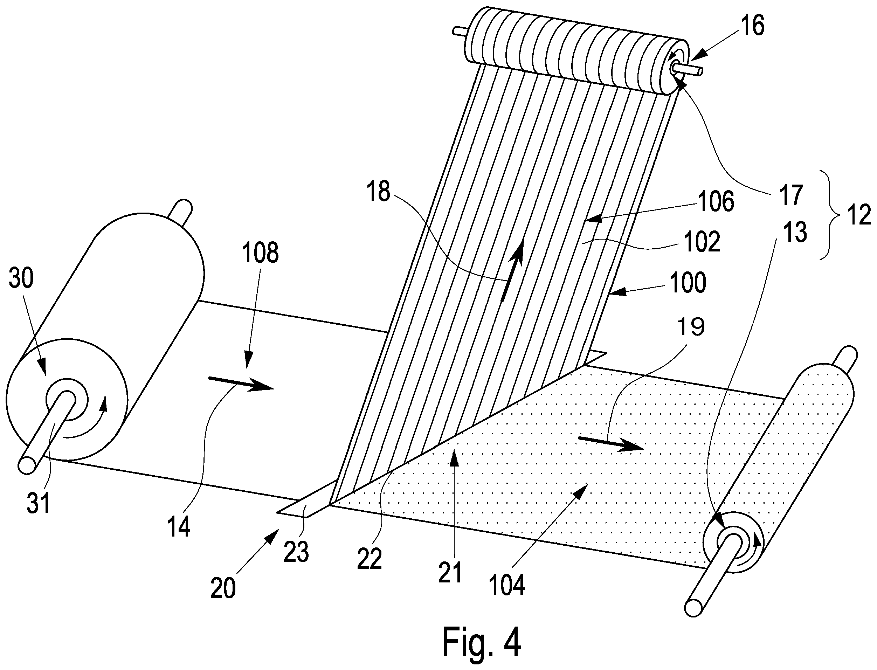

Fig. 4 zeigt eine weitere Vorrichtung zum Herstellen eines haftenden Folienverbunds 106, wobei die Ausführung im Wesentlichen der aus Fig. 3 entspricht.

Im Unterschied zu der Ausführung aus Fig. 3 sind in Fig. 4 die Haspeln 13; 17; 30 verdreht angeordnet, so dass auch die Drehrichtung der Haspeln 13; 17; 30 (dargestellt durch die Drehpfeile) in entgegengesetzter Richtung drehen.

## Patentansprüche

1. Verfahren zum Herstellen eines haftenden Folienverbunds (106) für ein transdermales therapeutisches System, mit den Schritten:
- Bereitstellen eines Laminats (108), wobei das Laminat (108) eine erste Folie (100), eine zweite Folie (104) und eine zwischen der ersten Folie und der zweiten Folie angeordnete, haftende Formulierung (102) aufweist,
- Zuführen des Laminats (108) in einer Zuführrichtung (14), und
- vorzugsweise abziehendes, Trennen der ersten Folie (100) mit darauf angeordneter Formulierung (102) von der zweiten Folie (106),
wobei das Trennen der Folien (100, 104) durch ein schräg zur Zuführrichtung (14) verlaufendes Abführen der ersten Folie (100) von der zweiten Folie (104) erfolgt,
wobei die zweite Folie (104) in Zuführrichtung (14) weitergeführt wird, und
wobei beim Abführen der ersten Folie (100) eine Drehung der ersten Folie (100) um deren Längsachse erfolgt,
wobei die Formulierung (102) einen Wirkstoff und/oder einen Klebstoff aufweist.

2. Verfahren nach Anspruch 1, **dadurch gekennzeichnet, dass** die erste Folie (100) in einem Winkel von über 60°, bevorzugt von 60° bis 120°, besonders bevorzugt von ca. 90°, relativ zur Zuführrichtung (14) abgeführt wird.

3. Verfahren nach Anspruch 1 oder 2, **dadurch gekennzeichnet, dass** die Drehung der ersten Folie (100) um deren Längsachse um mindestens 60°, bevorzugt um 90° bis 270°, besonders bevorzugt um ca. 180° erfolgt.

4. Verfahren nach einem der Ansprüche 1-3, **dadurch gekennzeichnet, dass** das Zuführen des Laminats (108) in einer Zuführebene, das Abführen der ersten Folie (100) in einer Abführebene, sowie vorzugsweise ein Weiterführen der zweiten Folie (104) nach dem Trennen in einer Weiterführebene erfolgt, wobei mindestens zwei der Zuführebene, der Weiterführebene und der Abführebene parallel, insbesondere identisch, zueinander sind.

5. Verfahren nach einem der Ansprüche 1 bis 4, **dadurch gekennzeichnet, dass** zumindest eine der Folien (100; 104) haftreduziert, vorzugsweise mit Silikon beschichtet, ist.

6. Verfahren nach einem der Ansprüche 1 bis 5, **dadurch gekennzeichnet, dass** die zweite Folie (104) eine geringere Haftung aufweist als die erste Folie (100); oder die erste Folie (100) eine geringere Haftung aufweist als die zweite Folie (104); oder die erste Folie (100) die zweite Folie (104) im Wesentlichen die gleiche Haftung aufweisen.

## Claims

1. A method for manufacturing an adherent film composite (106) for a transdermal therapeutic system, comprising the following steps:
- providing a laminate (108), the laminate (108) comprising a first film (100), a second film (104), and an adherent formulation (102) arranged between the first film and the second film,
- feeding the laminate (108) in a feeding direction (14), and
- separating, preferably in a peeling manner, the first film (100) with the formulation (102) arranged thereon from the second film (106),
wherein the films (100, 104) are separated by the first film (100) being fed out obliquely to the feeding direction (14) from the second film (104),
wherein the second film (104) is passed on in the feeding direction (14), and
wherein the first film (100) rotates about its longitudinal axis when feeding out the first film (100),
wherein the formulation (102) comprises an active ingredient and/or an adhesive.

2. The method according to claim 1, **characterized in that** the first film (100) is fed out at an angle of more than 60°, preferably from 60° to 120°, particularly preferred approx. 90°, relative to the feeding direction (14).

3. The method according to claim 1 or 2, **characterized in that** the rotation of the first film (100) about its own longitudinal axis is at least 60°, preferably 90° to 270°, and particularly preferred approx. 180°.

4. The method according to any one of claims 1-3, **characterized in that** the laminate (108) is fed in a feeding plane, the first film (100) is fed out in a feeding-out plane and, preferably, the second film (104) is passed on after separation in a passing plane, wherein at least two of the feeding plane, the passing plane and the feeding-out plane are parallel, in particular identical to one another.

5. The method according to any one of claims 1 to 4, **characterized in that** at least one of the films (100; 104) is adherence-reduced, preferably coated with silicone.

6. The method according to any one of claims 1 to 5, **characterized in that** the second film (104) has a lower adherence than the first film (100); or the first film (100) has a lower adherence than the second film (104); or the first film (100) and the second film (104) substantially have the same adherence.

## Revendications

1. Procédé de fabrication d'un film composite adhésif (106) pour système thérapeutique transdermique, comprenant les étapes consistant à :
- fournir un stratifié (108), où le stratifié (108) comprend un premier film (100), un second film (104) et une formulation adhésive (102) agencée entre le premier film et le second film,
- guider le stratifié (108) dans une direction d'amenée (14), et
- séparer, de préférence par pelage, le premier film (100) avec la formulation (102) disposée sur celui-ci du second film (106),
dans lequel la séparation des films (100, 104) s'effectue en détachant le premier film (100) du second film (104) en oblique par rapport à la direction d'amenée (14),
dans lequel le second film (104) est guidé ensuite dans la direction d'amenée (14), et
dans lequel le premier film (100) subit une rotation autour de son axe longitudinal pendant le détachement du premier film (100),
dans lequel la formulation (102) présente un principe actif et/ou une colle.

2. Procédé selon la revendication 1, **caractérisé en ce que** le premier film (100) est détaché selon un angle supérieur à 60°, de préférence de 60° à 120°, de manière particulièrement préférée d'environ 90°, par rapport à la direction d'amenée (14).

3. Procédé selon la revendication 1 ou 2, **caractérisé en ce que** la rotation du premier film (100) autour de son axe longitudinal s'effectue sur au moins 60°, de préférence sur 90° à 270°, de manière particulièrement préférée sur environ 180°.

4. Procédé selon l'une des revendications 1 à 3, **caractérisé en ce que** le guidage du stratifié (108) se fait dans un plan d'alimentation, le détachement du premier film (100) se fait dans un plan de détachement, et **en ce que** de préférence le guidage ultérieur du second film (104) après la séparation se fait dans un autre plan d'amenée, au moins deux des plan d'amenée, autre plan d'amenée et plan de détachement étant parallèles, en particulier identiques.

5. Procédé selon l'une des revendications 1 à 4, **caractérisé en ce qu'**au moins l'un des films (100 ; 104) est à adhérence réduite, de préférence revêtu de silicone.

6. Procédé selon l'une des revendications 1 à 5, **caractérisé en ce que** le second film (104) présente une adhérence inférieure à celle du premier film (100) ; ou le premier film (100) présente une adhérence inférieure à celle du second film (104) ; ou le premier film (100) et le second film (104) présentent sensiblement la même adhérence.
